(19) Europäisches Patentamt
European Patent Office
Office européen des brevets

(11) Veröffentlichungsnummer: **0 359 058**
**A1**

# (12) EUROPÄISCHE PATENTANMELDUNG

(21) Anmeldenummer: **89116158.0**

(22) Anmeldetag: **01.09.89**

(51) Int. Cl.⁵: **C07J 9/00 , A61K 31/575 , C07J 41/00 , A61K 47/28 , A61K 7/48**

(30) Priorität: **12.09.88 DE 3830932**

(43) Veröffentlichungstag der Anmeldung:
**21.03.90 Patentblatt 90/12**

(84) Benannte Vertragsstaaten:
**ES GR**

(71) Anmelder: **DIAMALT AG**
**Georg-Reismüller-Strasse 34**
**D-8000 München 50(DE)**

(72) Erfinder: **Messerschmitt, Thilo, Dr.**
**Unterübermoos 142**
**D-8098 Pfaffing(DE)**
Erfinder: **Mäke, Siegfried**
**Kufsteiner Strasse 109**
**D-8201 Raubling(DE)**
Erfinder: **Aigner, Arno, Dr.**
**Happinger Strasse 74**
**D-8200 Rosenheim(DE)**
Erfinder: **Vogt, Hubert, Dr.**
**König-Ludwig-Strasse 5 d**
**D-8201 Raubling(DE)**
Erfinder: **Bauer, Adolf, Dr.**
**Wendelsteinstrasse 6 C**
**D-8201 Raubling(DE)**

## (54) 2 -Hydroxy-N,N,N-trimethyl-ethanaminiumsalze von 5beta-Cholan-24-säure-Derivaten.

(57) 2-Hydroxy-N,N,N-trimethylethanaminiumsalze von 5β-Cholan-24-säure-Derivaten, die gekennzeichnet sind durch die allgemeine Formel I

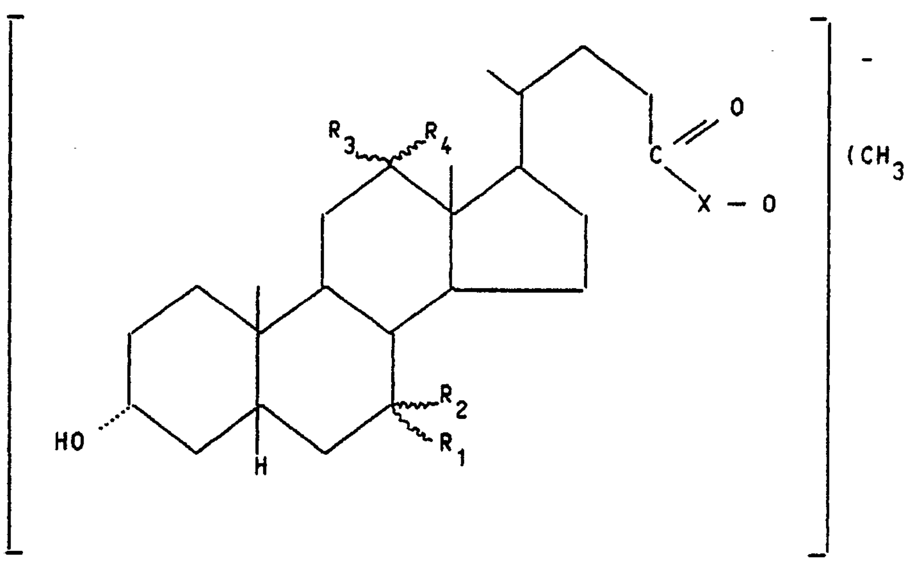

$(CH_3)_3\overset{+}{N}CH_2CH_2OH$

(I),

worin
$R_1$ und $R_2$ sowie $R_3$ und $R_4$ gemeinsam eine Oxogruppe oder zwei Wasserstoffatome ein Wasserstoffatom

EP 0 359 058 A1

und eine Hydroxygruppe bedeuten und
X eine C-O-Bindung oder eine Gruppierung der Formel $-NH-CH_2-CO-$ oder $-NH-(CH_2)_2-SO_2-$ darstellt, sind pharmakologisch und kosmetisch wirksame Verbindungen.

## 2-Hydroxy-N,N,N-trimethylethanaminiumsalze von 5β-Cholan-24-säure-Derivaten

Die Erfindung betrifft den in den Patentansprüchen gekennzeichneten Gegenstand.

2-Hydroxy-N,N,N-trimethylethanaminiumsalze ( = Cholinsalze) der allgemeinen Formel I gemäß Patentanspruch 1 sind pharmakologisch wirksame Substanzen die beispielsweise als Wirkstoffe von Cholagoga oder Gallenwegstherapeutika verwendet werden können.

Die Herstellung der 2-Hydroxy-N,N,N-trimethylethanaminiumsalze ( = Cholinsalze) der allgemeinen Formel I gemäß Patentanspruch 1 erfolgt unter Bedingungen, wie sie dem Fachmann wohlbekannt sind.

So kann man beispielsweise die freien Carbonsäuren der allgemeinen Formel II gemäß Patentanspruch 11 in einem polaren inerten Lösungsmittel mit Cholin umsetzen. Geeignete Lösungsmittel sind beispielsweise niedere Alkohole (Methanol, Ethanol, Propanol, Isopropanol etc.) oder niedere Ketone (Aceton, Methylethylketon). Andererseits sind auch dipolare aprotische Lösungsmittel (Dimethylformamid, Hexamethylphosphorsäuretriamid etc.) geeignet. Diese erbringen aber in der Regel keine Vorteile. Verwendet man als Lösungsmittel niedere Alkohole und etwa 0,2 bis 1 mol Säure pro mol Cholin, so kann man die Salze in einfacher Weise isolieren, indem man das Reaktionsgemisch eindampft und/oder die Reaktionsprodukte durch Zugabe unpolarer Lösungsmittel, wie zum Beispiel Aceton ausfällt.

Andererseits kann man auch die Alkalimetallsalze der Cholan-24-säure-Derivate der allgemeinen Formel II gemäß Patentanspruch 11 (vorzugsweise die Natrium-oder Kaliumsalze) mit Cholinsalzen anorganischer Säure (vorzugsweise Cholinchlorid oder Cholinsulfat) umsetzen. Diese Umsetzung wird ebenfalls in inerten polaren Lösungsmittel durchgeführt. Führt man die Reaktion in einem der oben genannten niederen Alkohole unter Verwendung etwa äquimolarer Mengen beider Reaktionspartner durch, so kann man das Reaktionsprodukt nach Abfiltrieren des Alkalimetallsalzes der anorganischen Säure in einfacher Weise isolieren, indem man das Filtrat einengt und/oder durch Zugabe unpolarer Lösungsmittel, wie zum Beispiel Aceton ausfällt.

Diese Reaktionsvariante läßt sich selbstverständlich auch in der Weise durchführen, daß man die freien Säuren der Formel II mit einem Cholinsalz einer anorganischen Säure in einem inerten Lösungsmittel, beispielsweise in Gegenwart von Alkalimetallhydrogenkarbonaten (Natriumhydrogenkarbonat oder Kaliumhydrogenkarbonat) umsetzt.

Die 2-Hydroxy-N,N,N-trimethylethanaminiumsalze der allgemeinen Formel I gemäß Patentanspruch 1 besitzen die gleiche pharmakologischen Wirkung wie die 5β-Cholan-24-säure-Derivaten der allgemeinen Formel II gemäß Patentanspruch 10. Darüberhinaus zeigen sie eine bakterizide oder virizide Wirksamkeit.

Die erfindungsgemäßen Verbindungen zeichnen sich aber unter anderen durch eine gute Wasserlöslichkeit, eine gute Stabilität, eine relativ geringe Basizität, eine relativ gute Resorbierbarkeit aus.

Zur Herstellung von Arzneimittelspezialitäten werden die erfindungsgemäßen Verbindungen gegebenenfalls mit den üblichen Zusätzen und Geschmackskorrigentien zu Tabletten, Dragees und Kapseln verarbeitet, die normalerweise 50 bis 500 mg Wirkstoff pro Dosiseinheit enthalten.

Wenn die Arzneimittelspezialität zur medikamentösen Auflösung von Cholesterin-Gallensteinen zur Prophylaxe der Gallensteinbildung bzw. als bakterizide oder virizide Mittel dienen soll, werden dem Patienten im allgemeinen 5 bis 20 mg Wirkstoff pro kg Körpergewicht und Tag verabreicht.

Die hohe Wasserlöslichkeit der erfindungsgemäßen Substanzen erlaubt darüber hinaus auch die Herstellung wässriger Lösungen, die beispielsweise zur Infusion oder Injektion geeignet sind, welche man beispielsweise zur Behandlung von primärer biliärer Zirrhose und primärer selerosierender Cholangitis sowie auch zur Behandlung von organbedingten akuten Störungen des Gallenflusses und der Darmresorption vorteilhaft anwenden kann, oder welche zur Spülung des Gallengangs oder der Gallenblase mittels percutan transhepatischer Drainage geeignet sind.

Darüberhinaus eignen sich die erfindungsgemäßen Substanzen auch zur Verarbeitung in Wasser/Öl und/oder Öl/Wasser Emulsionen. Die so gebildeten Lotionen, Cremes oder Salben eignen sich als dermatologische Präparate beispielsweise zur Wundbehandlung oder als Kosmetika. Die Herstellung dieser Lotionen, Cremes oder Salben erfolgt nach Methoden, wie sie dem Fachmann wohl bekannt sind (Dr. J. Stephan Jellinek "Kosmetologie" 2. Auflage, 1967, Dr. Alfred Hüthig Verlag, Heidelberg (DE) und Europäische Patentschrift 0065 929).

Die erfindungsgemäßen Substanzen eignen sich ferner als galenische Hilfsmittel für pharmazeutische Zubereitungen von Wirkstoffen um deren transdermale Resorption zu verbessern.

Die nachfolgenden Ausführungsbeispiele dienen zur Erläuterung des erfindungsgemäßen Verfahrens.

Beispiel 1

21,6 g Natriumsalz der 3α,7β-Dihydroxy-5β-cholan-24-säure (= 0,052 mol) werden unter Erwärmen in 250 ml Ethanol gelöst, mit einer Lösung von 7,0 g Cholinchlorid in 50 ml Ethanol versetzt und 2 Stunden lang aufbewahrt. Dann läßt man die Reaktionsmischung erkalten, filtriert das ausgefallene Kochsalz ab und engt das Filtrat weitgehend ein und tropft den noch fließfähigen Rückstand in 0,5 l Aceton ein. Man rührt die Mischung noch 2 Stunden lang, saugt das Produkt ab und trocknet es im Vakuum. Man erhält so 17,9 g 2-Hydroxy-N,N,N-trimethylethanaminiumsalz der 3α,7β-Dihydroxy-5β-cholan-24-säure mit einem Schmelzpunkt von 185 bis 190° C.

Beispiel 2

6,05 g Cholin (= 0,05 mol) werden in 50 ml Ethanol gelöst, mit 19,6 g 3α,7β-Dihydroxy-5β-cholan-24-säure (= 0,05 mol) versetzt und nach erfolgter Lösung zur Trockne eingeengt. Der Rückstand wird in Aceton aufgenommen, das abgeschiedene Kristallisat aubgesaugt und getrocknet. Man erhält 21,6 g 2-Hydroxy-N,N,N-trimethylethanaminiumsalz der 3α,7β-Dihydroxy-5β-cholan-24-säure mit einem Schmelzpunkt von 186 bis 190° C.

Beispiel 3

6,05 g Cholin (0,05 mol) werden in 50 ml Ethanol gelöst, mit 19,6 g 3α,7α-5β-cholan-24-säure (= 0,05 mol) versetzt und nach erfolgter Lösung in 200 ml Aceton eingetropft. Man rührt noch eine Stunde lang, saugt das abgeschiedene Kristallisat ab und trocknet es im Vakuum. Man erhält so 20,0 g 2-Hydroxy-N,N,N-trimethylethanaminiumsalz der 3α,7α-Dihydroxy-5β-cholan-24-säure mit einem Schmelzpunkt von 70 bis 75° C.

Beispiel 4

11,7 g 3α,12α-Dihydroxy-5β-cholan-24-säure (= 0,030 mol) werden in 30 ml Ethanol gelöst und mit 14,3 ml (= 0,030 mol) einer 2,1 molaren Lösung von Cholin-Base in Ethanol versetzt. Dann tropft man die Reaktionsmischung in 250 ml Aceton ein, saugt den Niederschlag ab und trocknet ihn im Vakuum.

Man erhält so 12,8 g 2-Hydroxy-N,N,N-trimethylethanaminiumsalz der 3α,12α-Dihydroxy-5β-cholan-24-säure mit einem Schmelzpunkt von 253 bis 255° C.

Beispiel 5

57 g 3α,12β-Dihydroxy-5β-cholan-24-säure (= 0,145 mol) werden in 100 ml Ethanol gelöst und mit einer Lösung von 17,5 g Cholin-Base (145 mmol) in Ethanol versetzt. Dann destilliert man das Ethanol ab, versetzt den Rückstand mit 200 ml Aceton, saugt den Niederschlag ab und trocknet ihn im Vakuum.

Man erhält so 60 g 2-Hydroxy-N,N,N-trimethylethanaminiumsalz der 3α,12β-Dihydroxy-5β-cholan-24-säure mit einem Schmelzpunkt von 170 bis 174.5° C.

Beispiel 6

4,6 g N-(3α,7α-Dihydroxy-24-oxo-5β-cholan-24-yl)-glycin (= 0,00832 mol) werden in 10 ml Ethanol gelöst und mit einer Lösung von 1,0 g Cholin-Base (= 0,00832 mol) in 10 ml Ethanol versetzt. Dann tropft man die Mischung in 200 ml Aceton ein, rührt noch einige Zeit, saugt die Kristalle ab und trocknet sie im Vakuum.

Man erhält so 3,6 g 2-Hydroxy-N,N,N-trimethylethanaminiumsalz des N-(3α,7α-Dihydroxy-24-oxo-5β-cholan-24-yl)-glycins mit einem Schmelzpunkt von 80 bis 83° C.

Beispiel 7

8,04 g 2-[(3α,7β-Dihydroxy-24-oxo-5β-cholan-24-yl)-amino]-ethansulfonsäure (= 0,0133 mol) werden in

30 ml Ethanol gelöst und mit einer Lösung von 1,61 g Cholin-Base (= 0,0133 mol) in 16 ml Ethanol versetzt. Dann tropft man die Mischung in 200 ml Aceton ein, rührt noch einige Zeit, saugt die Kristalle ab und trocknet sie im Vakuum.

Man erhält so 6,9 g 2-Hydroxy-N,N,N-trimethylethanaminiumsalz der 2-[(3α,7β-Dihydroxy-24-oxo-5β-cholan-24-yl)-amino]-ethansulfonsäure mit einem Schmelzpunkt von 80 bis 85° C.

Beispiel 8

30 g 3α-Hydroxy-7-oxo-5β-cholan-24-säure (0,1 mol) werden in 200 ml Ethanol aufgeschlemmt und mit einer Lösung von 12,1 g Cholin-Base (0,1 mol) in 100 ml Ethanol versetzt. Dann destilliert man das Lösungsmittel im Vakuum ab, versetzt den Rückstand mit 500 ml Aceton, filtriert den Niederschlag ab und trocknet sie im Vakuum.

Man erhält so 37,7 g 2-Hydroxy-N,N,N-trimethylethanaminiumsalz der 3α-Hydroxy-7-oxo-5β-cholan-24-säure mit unscharfem Schmelzpunkt.
$[\alpha]_D^{20}$ = -11,2° (Ethanol).

**Ansprüche**

1. 2-Hydroxy-N,N,N-trimethylethanaminiumsalze von 5β-Cholan-24-säure-Derivaten, gekennzeichnet durch die allgemeine Formel I

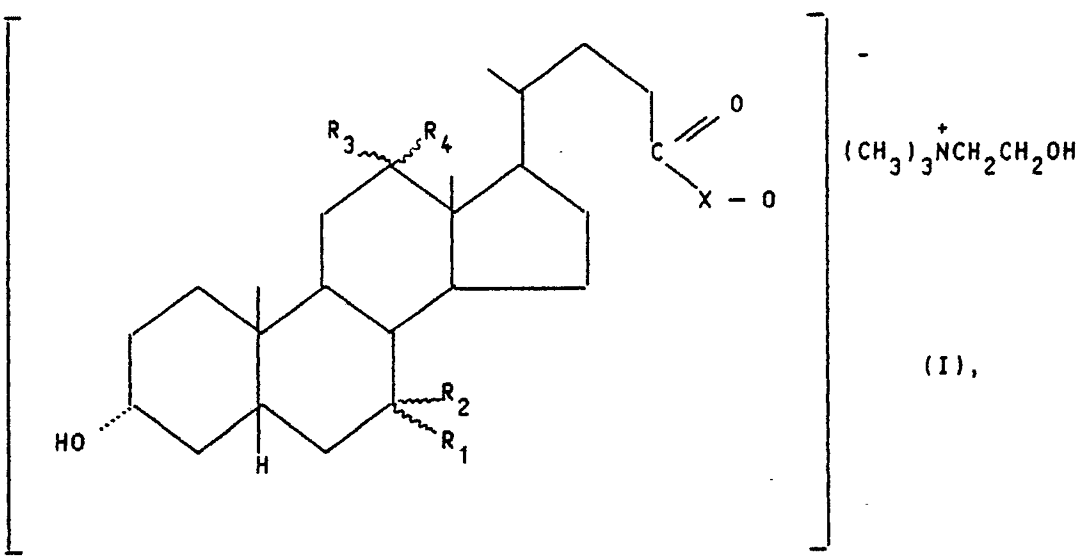

(I),

worin
$R_1$ und $R_2$ sowie $R_3$ und $R_4$ gemeinsam eine Oxogruppe, zwei Wasserstoffatome oder ein Wasserstoffatom und eine Hydroxygruppe bedeuten und
X eine C-O-Bindung oder eine Gruppierung der Formel
$-NH-CH_2-CO-$ oder $-NH-(CH_2)_2-SO_2-$ darstellt.

2. 2-Hydroxy-N,N,N-trimethylethanaminiumsalze von 5β-Cholan-24-säure-Derivaten gemäß Patentanspruch 1, dadurch gekennzeichnet, daß mindestens einer der Substituenten $R_1$ bis $R_4$ von Wasserstoff verschieden ist.

3. 2-Hydroxy-N,N,N-trimethylethanaminiumsalze von 5β-Cholan-24-säure-Derivaten der allgemeinen Formel I gemäß Patentanspruch 1, dadurch gekennzeichnet, daß die Substituenten $R_3$ und $R_4$ zwei Wasserstoffatome, eine Oxogruppe oder ein Wasserstoffatom und eine β-ständige Hydroxygruppe bedeuten.

4. 2-Hydroxy-N,N,N-trimethylethanaminiumsalze der 3α,7α-Dihydroxy-5β-cholan-24-säure.

5. 2-Hydroxy-N,N,N-trimethylethanaminiumsalze der 3α,7β-Dihydroxy-5β-cholan-24-säure.

6. 2-Hydroxy-N,N,N-trimethylethanaminiumsalze der 3α,12α-Dihydroxy-5β-cholan-24-säure.

7. 2-Hydroxy-N,N,N-trimethylethanaminiumsalze der 3α,12β-Dihydroxy-5β-cholan 24-säure.

8. 2-Hydroxy-N,N,N-trimethylethanaminiumsalze des N-(3α,7α-Dihydroxy-24-oxo-5β-cholan-24yl)-glycins.

9. 2-Hydroxy-N,N,N-trimethylethanaminiumsalze der 2-[(3α,7β-Dihydroxy-24-oxo-5β-cholan-24-yl)-amino]-ethansulfonsäure.

10. 2-Hydroxy-N,N,N-trimethylethanaminiumsalze der 3α-Hydroxy-7oxo-5β-cholan-24-säure.

11. Pharmazeutische Präparate enthaltend mindestens ein 2-Hydroxy-N,N,N-trimethylethanaminiumsalz der allgemeinen Formel I gemäß Patentanspruch 1.

12. Kosmetische Präparate enthaltend mindestens ein 2-Hydroxy-N,N,N-trimethylethanaminiumsalz der allgemeinen Formel gemäß Patentanspruch 1 bis 3.

13. Verfahren zur Herstellung von 2-Hydroxy-N,N,N-trimethylethanaminiumsalze der allgemeinen Formel I, gemäß Patentanspruch 1, dadurch gekennzeichnet, daß man in an sich bekannter Weise ein 5β-Chloan-24-säure-Derivat der allgemeinen Formel II

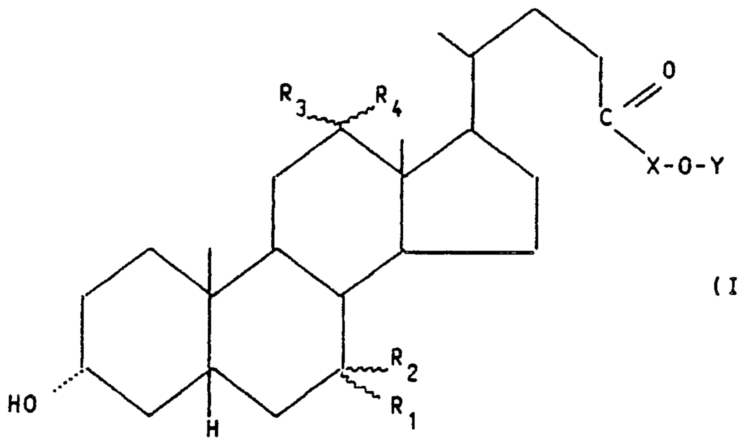

(II),

worin
$R_1$, $R_2$, $R_3$, $R_4$ und X die im Anspruch 1 genannte Bedeutung besitzen und
Y ein Wasserstoff oder ein Alkalimetallatom darstellt, mit einer Verbindung der allgemeinen Formel III

$$(CH_3)_3-\overset{Z}{\overset{|}{N}}-HC_2-CH_2-OH$$

worin
Z eine Hydroxygruppe darstellt, falls Y ein Wasserstoffatom ist, oder den Rest einer anorganischen Säure bedeutet, falls Y ein Alkalimetallatom ist, umsetzt.

Europäisches Patentamt

# EUROPÄISCHER RECHERCHENBERICHT

Nummer der Anmeldung

EP 89 11 6158

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | DE-C- 593 258 (EGON GLÜCKSMANN)<br>* Insgesamt *<br>--- | 1-3,6,<br>12 | C 07 J 9/00<br>A 61 K 31/575<br>C 07 J 41/00<br>A 61 K 47/28<br>A 61 K 7/48 |
| X | US-A-2 589 840 (A.E. MEYER)<br>* Insgesamt *<br>--- | 1-3,6,<br>12 | |
| X | FR-A-2 074 736 (R. PRUGNAUD)<br>* Insgesamt *<br>----- | 1-3,12 | |
| | | | **RECHERCHIERTE SACHGEBIETE (Int. Cl.5)** |
| | | | C 07 J 9/00<br>C 07 J 4/00<br>A 61 K 47/00 |

Der vorliegende Recherchenbericht wurde für alle Patentansprüche erstellt

| Recherchenort | Abschlußdatum der Recherche | Prüfer |
|---|---|---|
| DEN HAAG | 18-10-1989 | HENRY J.C. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)